# EUROPEAN PATENT SPECIFICATION

(11) **EP 2 037 855 B1**
(45) Date of publication and mention of the grant of the patent: **04.03.2015**
(21) Application number: 06747984.0
(22) Date of filing: 30.06.2006
(51) Int. Cl.: A61F 13/513, A61F 13/512, A61L 15/60

(54) **ABSORBENT ARTICLE**
SAUGFÄHIGER ARTIKEL
ARTICLE ABSORBANT

(43) Date of publication of application: 25.03.2009
(73) Proprietor: SCA HYGIENE PRODUCTS AB, 405 03 Göteborg (SE)
(72) Inventor: BOISSIER, Elisabeth, S-434 94 Vallda (SE)
(74) Representative: Zacco Sweden AB
(86) International application number: PCT/SE2006/000817
(87) International publication number: WO 2008/002219

(56) References cited:
- WO-A1-00/24351
- WO-A1-99/42068
- WO-A1-03/015681
- GB-A- 1 414 709
- GB-A- 2 284 786
- US-A- 3 585 998
- US-A1- 2002 058 056
- US-A1- 2004 170 589
- US-A1- 2005 154 362
- US-A1- 2006 069 370
- US-B1- 6 461 339

## Description

### TECHNICAL FIELD

The invention relates to an absorbent product having an extent in a plane in the longitudinal direction and the lateral direction and a thickness perpendicular to the plane when the product is positioned in a plane position. The absorbent product consists of a backing layer, a top layer and between them an absorption body. The absorption body has a first surface and a second surface, the backing layer being arranged over the second surface of the absorption body. The product comprises in the longitudinal direction a rear section, a front section and between them a central section, and exhibits in the lateral direction a first lateral zone, a second lateral zone and positioned between them a central zone.

### BACKGROUND ART

In previously disclosed absorbent products, the top layer comprises a layer of material that is liquid-permeable in order to be able to transport liquid from the top layer to the subjacent absorption body. The liquid can be menstrual fluid or urine, and the choice of the material used for the top layer depends on which of these liquids the absorbent product is primarily intended to take up. When choosing the material, consideration must also be given to the required suitability of the top layer having regard for the need for the wearer to experience the material as comfortable against his/her body, and for the wearer to experience a sensation of dryness after the liquid has been excreted. A hydrophobic material is already known to give such a dry sensation. Also previously disclosed is the embodiment of a water-impermeable layer of material with holes in order to make the top layer water-permeable, at the same time as the water-impermeable material in other respects prevents the transport of liquid from the absorption body.

The problems associated with the choice of material for the top layer are particularly great in the lateral zones, because the lateral zones of the absorbent product make contact at least partially with a wearer's groin. The nature of the problem is that the groin moves relative to the lateral zones, which can give rise to chafing, and that the lateral zones become crumpled. Movement by the wearer also causes the absorbent product to describe simultaneous movement in the lateral zones, which imposes high demands on the material in order for the material not to rupture. The movement in the lateral zones is usually less than the movement in the central zone, which can give rise to chafing on the wearer if an inappropriate material is used in the lateral zones.

WO 03/015681 depicts an absorbent product comprising a top layer of a film or nonwoven containing a number of holes, the holes being larger in the central zone than in the lateral zones. The small holes in the lateral zones are provided to give the material a softer feel compared with a non-perforated material. One problem associated with holes is that the holes are always open and permit excessive reflux. Another problem is that the holes permit the leakage of material that is lying loosely under the top layer, for example superabsorbents (SAP), odour inhibitors in the form of granulates and loosely lying fiber material. Superabsorbents are small balls or granules of an absorbent material lying loosely in the absorption body, which can be transformed into a gel-like substance by the absorption of water. The loosely lying material that has found its way out through the holes ends up between the absorbent product and the wearer's skin and give rise to discomfort for the wearer, for example in the form of chafing. The problems mentioned above are especially great in the lateral zones of the absorbent product, because the movement in the lateral zones influences the absorption body in such a way that SAP and liquid exhibit a tendency to be transported out through the holes by the movement of the lateral zone. Making the holes so small that the superabsorbents are not able to leak out through the holes, or so small that the moisture reflux problem is reduced, would reduce the transport of liquid from the top layer to the absorption body, that is to say the admission capacity of the top layer to the subjacent material, to an unacceptably low level. Similar problems exist for all particles that are subject to the risk of leaking out through the holes, for example the above-mentioned particles.

A wish and a need accordingly remain for an absorbent product, in which the above problems are overcome by means of a top layer having improved characteristics in the lateral zones as far as maximum liquid-permeability, minimal reflux and minimal leakage of loosely lying material are concerned, at the same time as the top layer is provided with a soft and comfortable surface for the wearer when the wearer moves.

### DISCLOSURE OF INVENTION

The invention relates to an absorbent product according to claim 1

The wet area is the area of the absorbent product which is intended to be wetted first by excreted bodily fluid, and which essentially coincides with that part of the absorbent product that is formed by the section of the central zone and the central section, and which can vary in size and position depending on the principal area of application of the absorbent product, for example as a light incontinence pad or sanitary towel.

The subdivision of the absorbent product into zones and sections is of a theoretical nature without distinct boundaries and is made for the sole purpose of facilitating an appreciation of the invention. The laterally and longitudinally extending lines can thus be straight or curved, depending on the shape of the product. The wet area is thus not restricted to having a square or rectangular geometry. It may be mentioned here by way of example that the wet area can have any appropriate geometry, for example circular, oval, triangular, etc. The peripheral part containing slits in accordance with the invention, that is to say the part of the top layer which at least partially encloses the wet area, thus has a corresponding shape at the interface with the wet area. The position of the slits is thus able to follow the contours of the wet area in the lateral zones, the rear section and the front section, and it is not restricted to positions within the area described above that is defined by the lateral zones, the rear section and the front section, excluding the section of the central zone and the central section. In a corresponding manner, the lateral zones, the rear section and the front section follow the contours of the absorbent product, that is to say they follow the edge part of the absorbent product.

When using the absorbent product as an incontinence pad, the wet area consists of the part of the absorbent product which, when it is being used by a wearer, lies facing towards and in close proximity to the wearer's urethra. The wet area thus constitutes the part of the absorbent product that will receive the largest flow of liquid in conjunction with the excretion of liquid waste by the wearer. In the case of a sanitary towel, the centre of the wet area can be displaced somewhat in relation to the urethra in such a way that the wet area is positioned against that part of a women's lower abdomen from which the flow of menstruation fluid is discharged.

The absorbent product is a panty liner or a sanitary towel, which can be used as undergarment protection in conjunction with menstruation or light incontinence or to protect the undergarments from discharges, etc.

The expression slit is used here to denote an essentially one-dimensional through going opening in a layer of material having an extent in the longitudinal direction and a depth which depend on the thickness of the layer of material and the manner in which the slit is executed in the material. The slit comprises two boundary surfaces, each delimited by the extent of the slit in the longitudinal direction and the thickness of the layer of material. Since the layer of material is unaffected by external forces, the boundary surfaces are in contact with one another and close the opening in the same way as if a slit were not to be present, but with the difference that the bonds which otherwise hold the layer of material together are absent in the section of the layer of material in which the slits are present. The slits are manufactured by rupturing the bonds in the layer of material over the distance required for the extension of the slit. The bonds can be ruptured, for example, by producing an incision in the layer of material by means of slitting, cutting or in some other way. This is entirely true in a theoretical model, although in practical reality material will disappear on a micrometric scale as a consequence of, for example, blunt tools, etc. The loss of material must not be confused with the loss of material when making holes, where the opening is not one-dimensional, but rather two-dimensional, due to the requirement in the case of holes for material to be removed in such a way that a permanent opening is formed, where corresponding boundary surfaces are not able to make contact with one another if the layer of material is not influenced by external forces. In its uninfluenced state, the slit thus exhibits an extent in one direction and has the same thickness as the material.

The definition of slits versus holes is important, because only slits offer the advantages that are sought by the present invention. Unlike holes, slits remain closed up to the point in time at which the layer of material is influenced by external forces, when the slits are caused to open by the interfaces being displaced in relation to one another.

This advantage is utilized in the invention in that the slits in the absorbent product before use are essentially closed and are able to retain substances that have been placed under the top layer in the lateral zones. Examples of substances are superabsorbents (SAP) and lotion. When the product is used, the slits are influenced by the movement of the wearer in such a way that they are opened and closed, which minimizes the risk of SAP leaking out. SAP can thus be laid freely under the top layer without SAP leaking out from the product. Because the slits are opened and closed, it is also possible for lotion to be laid under the top layer without the risk of the absorbent product being made sticky by leaking lotion before use, although during use lotion is released via the slits onto the areas of the wearer's skin that are subject to friction.

Another advantage of a slit is that the layer of material is breathable and liquid-permeable in precisely the same way as when a hole is made in the material, including if the layer of material without slits is airtight and watertight, but with the difference and the advantage that any liquid that has been absorbed by the subjacent absorption body finds it more difficult to pass through the layer of material because the slits are open and closed alternately, whereas a hole is always open and in this way does not restrict the reflux of liquid from the absorption body.

The slits in the open state possess a larger open area than the area that a circular hole would possess if the hole had been made so small that SAP was not able to find its way out. One advantage of the slit is that the larger opening in the slit is elongated and possesses a smaller maximum opening width than a corresponding radius in the hole, which gives good liquid-permeability without SAP being able to penetrate out through the opening, and minimal reflux from the absorption body, as well as a soft feeling against the wearer's skin. Furthermore, a larger open area provides better transport of the air through the top layer, that is to say the ability of the top layer to breathe is increased.

The slit has a maximum opening length that is larger than the SAP particle, which theoretically would provide the SAP particle with the ability to pass through the opening, although the risk of the SAP particle being able to pass through the opening is reduced because the slit is only open intermittently. One effect of this is that the sits in the open state can be permitted to have an even larger open area, with the advantage that the transport of liquid to the absorption body increases, and the breathability of the material increases.

The top layer comprises one or a plurality of layers of material and is able, before slitting takes place, to be either liquid-permeable or water-tight, or vapour-tight, or vapour-permeable, or air-tight, or air-permeable, or to have an appropriate combination of the various characteristics. The top layer can consist of a woven or nonwoven textile material, a plastic material, a fiber material or a combination of the various materials, or some other appropriate material.

The backing layer is a moisture barrier layer which can be vapour-permeable or vapour-tight. The backing layer can consist of a treated textile material, or a plastic material, or a fiber material or a combination of these materials.

The top layer can comprise holes or slits, including in the central zone of the product. The central zone is not influenced by the movement of the wearer in the same way as the lateral zones, and entirely different conditions apply there for this reason. Holes can thus be present in the central zone, but not in the lateral zones, for the reasons given above.

The expression movement of the user is used here to denote the movement performed by the wearer in the event of a change in the position of his/her legs, when the inside of the thighs influences the lateral zones of the absorbent product. Such a change in the position of the inside of the thighs relative to the absorbent product occurs in both a sitting, standing, reclining and crouching position, etc., and when the wearer crawls, creeps, walks, runs, jumps, cycles, swims, etc. Even a very small change in position in the lateral zones is sufficient for the slits to be opened and closed in accordance with the invention, for which reason every movement by the wearer in principle gives rise to a movement in the lateral zones.

The absorbent product relates to protection for menstruation and light incontinence. The invention is not intended for diapers or incontinence pads of the panty type, because such an absorbent product has a different construction from means of protection such as sanitary towels, panty liners and incontinence pads. The absorbent product in accordance with the invention can essentially be accommodated in a normally sized panty and does not have the front, rear and side panels that are present in diaper pants and which form leg openings and a waist part respectively.

According to the invention, the top layer comprises slits only in the lateral zones, and these can be restricted, furthermore, to only the central section of the lateral zones. The advantages of the slits described above by comparison with holes are especially advantageous in the lateral zones and in the central section of the lateral zones, because the absorbent product is influenced when it is being worn to an especially high degree by the movement of the wearer in these areas. The slits thus open and close at a greater frequency in the lateral zones than in the front section and the rear section, for example. The invention is not restricted to this area, however, but in accordance with another embodiment of the invention the top layer comprises slits in the lateral zones in the rear section and/or in the front section and/or in the central section. The slit areas thus partially enclose the wet area in these embodiments.

The manner in which the slits extend in the top layer depends on a number of factors, such as the direction of movement of the web of material during the slitting operation and the choice of material for the top layer. It can be mentioned here by way of example that a slit will open when it is subjected to forces that act at an angle towards the direction in which the slit extends. The natural tendency for the slit to open is at its greatest when the forces act upon the slit in a direction oriented at 90° to the direction in which the slit extends. The top layer is manufactured in a web of material having a movement in a machine direction which usually coincides with the longitudinal direction of the absorbent product or its lateral direction. In conjunction with its manufacture, the web of material is influenced by forces in the machine direction which cause slits which lie perpendicular to the machine direction to be influenced to a maximum extent by these forces. The forces involved in this case can cause the material to split at the slits or, at any rate, can cause the slits to open essentially permanently. What is more, the finished absorbent product will contain slits having an extent either in the longitudinal direction or in the lateral direction, which will mean that the slits are affected essentially only by forces acting in one direction. If the slits are instead oriented at an angle to the machine direction, there is a smaller risk of the top layer splitting during manufacture. From the point of view of the product, a significant advantage is achieved in that a shape and an extent are imparted to the slits that are affected by forces both from the lateral direction and from the longitudinal direction and at angles in between. The comparisons indicated above apply to a slit with a given length. The fact that the slits are affected by forces in the lateral direction and in the longitudinal direction, and at angles in between, means that the natural tendency of the slits to open and close as the wearer moves will increase, because movement by the wearer will give rise to forces both in the lateral direction and in the longitudinal direction and in directions in between.

The slits themselves can be straight, S-shaped, V-shaped, Z-shaped, U-shaped, or can possess any other suitable shape. The slits can also comprise combinations of different shapes, for example a plurality of straight or curved slits arranged consecutively and having the same or a different length, where every other slit is oriented at an angle (preferably essentially 90°) in relation to the essential longitudinal extent of the preceding slit, but where the slits are situated at a distance from one another. The slits are thus arranged at an angle of between 0 and 180° relative to a longitudinally extending centre line, preferably in the range from 20°-65° and/or 110°-155° in relation to the longitudinally extending centre line. Different parts of the slits can have a different direction in relation to the centre line.

In order to describe the slit in more detail, the following description is based on a top layer having an extent in a single plane. It must be pointed out, however, that the absorbent product, when it is being worn, has a three-dimensional extent that is adapted in accordance with the wearer's body. The slit can be made in the top layer by means of a through going incision in the top layer perpendicular to the plane surface, but it can also be made in the top layer by means of a through going incision at an angle to the plane surface. In the latter case, the opposing edge parts of the slit overlap one another and change the characteristics of the slit in such a way that the top layer is air-permeable and water-permeable as a result of the overlapping parts being raised at an angle towards the plane surface, at the same time as a material lying loosely under the top layer finds it more difficult to escape through the slit than in the case of a slit with a straight incision, because the loose material is obliged to make its way through a longer channel than in the case of a straight incision. Furthermore, the overlapping parts permit air and water to diffuse out through the closed slit, at the same time as the material lying loosely on the under side of the top layer is prevented from finding its way out because it is too large to be able to diffuse out. A further advantage of a slit with inclined opposing edges is that a small lateral displacement, that is to say in the direction in which the plane surface extends, does not cause the slit to open, because the overlapping parts cover one another for the entire length of the opposing edge parts, in conjunction with which the material lying loosely is also restrained in the event of a quite small movement in the material. The top layer is relatively thin, for which reason the maximum movement of the lateral displacement must be small, although the advantages indicated above are also available when the lateral displacement exceeds the maximum movement, because the slit is caused to open less than in the case of a straight incision. It must be pointed out that in the case of a straight slit, however, air, water vapour and, where appropriate, water can also diffuse out through an opened slit before the slit has been widened to such an extent that the loose material is able to find its way out.

The absorption body is appropriately manufactured from a suitable fiber material in the form of natural or synthetic fibers having absorbent properties, or a mixture of natural fibers and synthetic fibers or other absorbent materials of a previously disclosed kind that are suitable for use in sanitary towels, incontinence pads and panty liners, for example. The absorption body can also contain a predetermined proportion, for example 20-60%, of superabsorbent materials, that is to say polymer materials in the form of particles, fibers, flakes or similar, which have the capacity to absorb and to chemically bind liquid equivalent to several times their own weight while forming an aqueous gel. This provides a very high water-absorbent capacity in the finished product.

It must also be noted that the absorption body can exhibit different forms, for example an essentially elongated and rectangular form, or alternatively some other more irregular form, for example hourglass or triangular form. The absorption body also has preferably rounded edges.

The liquid-permeable top layer appropriately consists of one or more layers of one or more of the following materials: a fibrous material, for example a soft nonwoven material, plastic film, mesh, open-celled foam, material laminate, etc. The top layer is preferably fully or partially perforated, that is to say slits are made in the top layer in accordance with the above, and holes can be present in the wet area. The top layer can appropriately consist of a perforated plastic film, for example a thermoplastic plastic material such as polyethylene or polypropylene, or a mesh-like layer of synthetic or textile material. Synthetic mono-, bi-, or multi-component fibers, made of polymers such as polyethylene, polypropylene, polyester, nylon or the like, are preferably used as a nonwoven material. Mixtures of different types of fibers can also be used for the aforementioned nonwoven material. The invention is not, however, restricted in principle to use only for top layers which consist of nonwoven material, but can also be applied in conjunction with the processing of other materials, for example films made of thermoplastics such as polyethylene or polypropylene.

The invention can also be implemented with a top layer which consists of different types of laminates or combinations of laminates and/or single layers. For example, the top layer can consist of a number of different laminates or single layers which cover parts of the surface of the product. In the event that the product consists of a plurality of laminates or single layers, for example subdivided into a plurality of longitudinal parts having different sections, these different sections can consist of different materials and can possess different characteristics. For example, each section can then have different types of perforation, hole positioning, dimensions, hydrophobicity, etc. The different sections can be joined together by means of ultrasonic welding in a previously disclosed manner that is not described here in detail.

The liquid-permeable top layer is preferably manufactured from a material that exhibits characteristics such as dryness and softness during the time when the absorbent product is being worn, because this top layer is in contact with the wearer's body. It is also desirable for the top layer to have a soft and textile-like surface which remains dry, even in the event of repeated wetting. The top layer can consist of a nonwoven material, for example, with a soft and smooth surface, such as a spunbond material made from polypropylene fibers. A perforated, hydrophobic nonwoven material may be used in order to permit the surface that is closest to the wearer's body to be kept dry, in conjunction with which holes are formed in the material that are larger than the distance between the fibers in the material. In this way, liquid can be led down through the holes in the top layer to the subjacent absorption body. Other examples of materials for the top layer are perforated plastic films such as a perforated polyester film. The top layer can be joined together with the subjacent backing layer and the absorption body, for example by means of adhesive, ultrasonic jointing or by means of some form of thermal bonding.

The top layer can also be a three-dimensional laminate of nonwoven and plastic film or a carded, thermally bonded material based 100% on polypropylene. The plastic film can be hydrophilic, pre-perforated (with small holes) and manufactured from a mixture of polyethylene and polypropylene. The nonwoven materials can have a weight per unit area in the range from 12-100 gsm, and in particular in the range from 15-60 gsm.

The nonwoven part of the top layer can also be a spunbond nonwoven material, an air-thru nonwoven material, a spunlace nonwoven (hydroentangled) material, a meltblown nonwoven material, or a combination of these. The raw material can be polypropylene (PP), polyethylene (PE) polyester (PET), polyamide (PA), or a combination of these. If a combination is used, this can be a mixture of fibers from different polymers, although each fiber can also contain different polymers (for example PP/PE bicomponent fibers or PP/PE copolymers). Where appropriate, the plastic film can consist of PE or PP, PET, PLA or amyl (or any other thermoplastic polymer), or a mixture or copolymers of the aforementioned polymers.

The perforated top layer can also be manufactured from a single layer of material, such as a nonwoven material or a film (as described above).

The holes in the top layer can be oval and slightly elongated in the direction of the machine. The holes can be round/circular or oval in the direction of the machine or the transverse direction. The holes in the wet area can also be replaced by slits, which by definition differ from the holes in that the slits do not constitute constant openings, but instead are through going incisions in the layer of material. The slits are opened and closed by movement in the material.

According to the invention, the slits are from 2 mm up to 15 mm in length, and preferably lie in the range from 3-10 mm. The length of the slits is measured along the boundary surfaces of the slits in a direction essentially perpendicular to the thickness of the top layer and when the slit is in its closed state.

The slits are arranged in the top layer with a mutual distance between the slits having a size in the order of 5-15 mm, although this is dependent on a range of factors, for which reason the distance between the slits can vary depending, among other things, on the material in the top layer and the length of the slits and the direction of the slits. This distance between the slits must be sufficiently great to prevent the top layer from being torn apart when the wearer moves, and sufficiently great to allow the slits to close in the desired manner under the influence of other slits, although at the same time sufficiently small for the ability to breathe and the liquid permeability to remain at an acceptable level. The durability of the top layer is largely governed, however, by the relationship between the surface containing slits and the surface without slits for a given material strength, where the distance between the slits is a subset of the parameters for the durability. The length of the slits and the distance between the slits and the direction of the slits vary depending on the material in the top layer, because the natural tendency of the slits to open depends on the characteristics of the material present in the top layer.

The backing layer is preferably liquid-impermeable (or at least possesses high resistance to penetration by liquid) and is thus so arranged as to prevent any leakage of excreted fluid from the product. The backing layer, on the other hand, may be executed so that it is vapour-permeable. For this purpose, the backing layer may be manufactured from a liquid-impermeable material which consists appropriately of a thin and liquid-proof plastic film. For example, plastic films of polyethylene, polypropylene or polyester can be used for this purpose. Alternatively, a laminate of nonwoven and plastic film or other suitable layers of material can be used as a liquid-proof backing layer. In a previously disclosed manner, the under side of the backing layer can be provided with beads of adhesive or some other previously disclosed attachment means, which can then be utilized for the application of the product to an item of clothing. The product can also be provided with wings, that is to say folding flaps which, in a previously disclosed manner, are arranged along the sides of the product and can be utilized in conjunction with the application of the product.

The product also includes a further layer of material in the form of a receiving layer (also referred to as an acquisition layer, an admission layer and a distribution layer, depending on the function of the material). The receiving layer can be in the form of a wadding material having an appropriately specified thickness and resilience, which is intended to be positioned between the absorption body and the top layer. The receiving layer possesses essentially the same dimensions as the top layer, with the exception of its thickness, however, which can deviate from the thickness of the top layer. It is also possible to establish that the receiving layer can consist of materials other than wadding material. For example, it may consist of a so-called airlaid material, which is usually based on cellulose fibers. The receiving layer can also incorporate fibrous materials in order to impart an appropriately balanced rigidity to it. The receiving layer can also incorporate an appropriate quantity of thermoplastic fibers in order to permit ultrasonic welding.

The receiving layer can appropriately be a porous, elastic, relatively thick layer of material, for example in the form of a fibrous wadding material, a carded fiber wadding, a tow material, or some other kind of bulky and/or resilient fiber material with a high instantaneous liquid intake capacity that is capable of storing liquid temporarily before it is absorbed by the subjacent absorption body. The receiving layer can also be in the form of a porous foam material. It can also consist of two or more layers of material. According to a preferred embodiment, the receiving layer can extend towards the lateral edges of the product, that is to say it possesses essentially the same form as the top layer. In this way, advantages can be achieved in respect of liquid distribution, edge sealing, etc.

When manufacturing the absorbent product, the top layer is joined to the backing layer and can also be joined to the receiving layer and/or the absorption body. Joining can take place by gluing; or by welding in the form of ultrasonic or laser; or by mechanical joining, for example in the form of embossing or compression, etc., or by some other appropriate method of joining, for example thermal bonding.

In a preferred embodiment, at least the wet area comprises a perforated two-dimensional or three-dimensional plastic or nonwoven film, where the rest of the top layer comprises a slit, nonwoven in the lateral zones and, where appropriate, in the front section and/or in the rear section of the central zone.

It must be stated, however, that the choice of material and the thickness and density of the layer of material may change in the future in the event of changed manufacturing methods and new material combinations, as a consequence of which the invention is not restricted to the materials and material combinations indicated above.

### BRIEF DESCRIPTION OF DRAWINGS

The invention is described below in conjunction with a number of Figures, in which:
Figure 1 depicts schematically a view of the top layer of an absorbent product in accordance with the invention;
Figure 2 depicts schematically a sectional view along the line II-II in Figure 1;
Figure 3 depicts schematically an enlarged view of section A in Figure 2;
Figure 4 depicts schematically an absorbent product according to Figures 1-3 when being worn against the lower abdomen of a wearer;
Figure 5a depicts schematically a slit according to the invention together with an SAP granule, and where;
Figure 5b depicts schematically a hole according to the prior art together with an SAP granule.

### MODE(S) FOR CARRYING OUT THE INVENTION

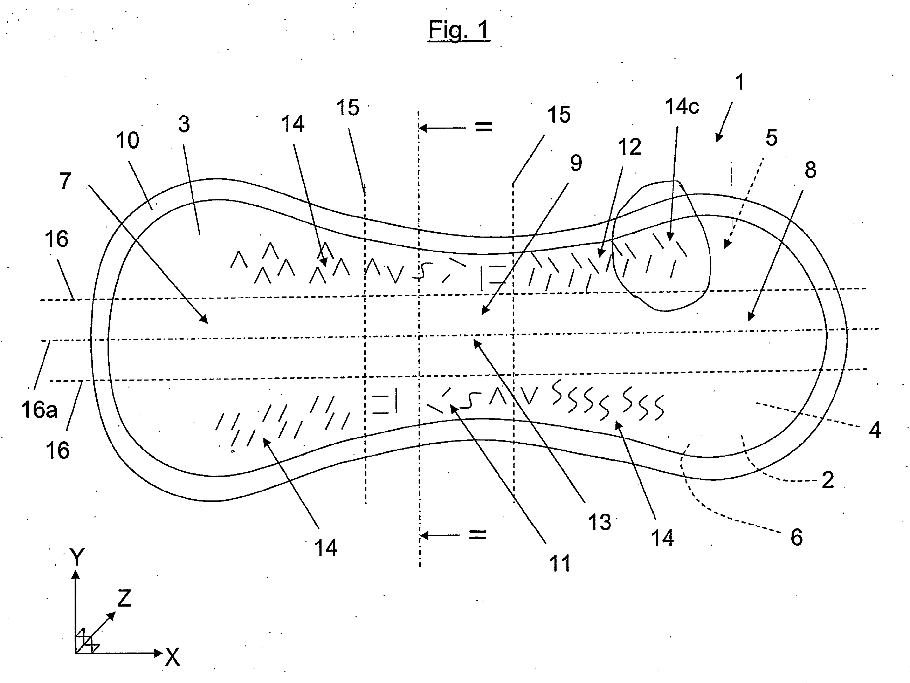

Figure 1 depicts schematically a view of the top layer 3 of an absorbent product 1 according to the invention. The absorbent product 1 here consists of a sanitary towel, which can be used as undergarment protection in conjunction with menstruation or light incontinence. The absorbent product 1 is positioned in a plane position and exhibits in this plane an extent in the longitudinal direction, the X-axis in the Figure, and in the lateral direction, the Y-axis in the Figure, and has a thickness perpendicular to the plane, the Z-axis in the Figure.

The absorbent product 1 comprises a backing layer 2, a top layer 3 and between them an absorption body 4. The absorption body 4 has a first surface 5 and a second surface 6, in conjunction with which the backing layer 2 is arranged over the second surface 6 of the absorption body 4, and the top layer 3 is arranged over the first surface 5 of the absorption body 4. The absorbent product is subdivided theoretically in the longitudinal direction into a rear section 7, a front section 8 and between them a central section 9. The absorbent product 1 is delimited by a peripheral edge part 10. The top layer 3 is described from the edge part 10, in the lateral direction, by a first lateral zone 11 and a second lateral zone 12 and positioned between them a central zone 13. The top layer 3 comprises in the lateral zones 11, 12 a layer of material containing slits 14. The slits 14 can have any desired embodiment, on condition that the slit 14 performs in the intended manner during use, that is to say the slit 14 opens and closes when the wearer moves. Figure 1 shows a number of embodiments of slits 14, namely curve-shaped, angle-shaped, diagonal, straight, and mixtures of the various types. The various types of slits 14 are shown in the same absorbent product 1 only for the purpose of enabling the simple illustration of different types of slits 14. The invention is not restricted to comprising different types of slits 14, but can be executed with a number of similar slits 14, or with a number of optional combinations. Depicted in Figure 1 are two laterally extending lines 15, which divide the product in the longitudinal direction in accordance with the above, and two lines 16 extending in the longitudinale direction, which divide the product in the lateral direction in accordance with the above. The direction in which the slits 14 extend differs appropriately, at least partially, from the longitudinal direction of the product and from the lateral direction of the product, because such an arrangement of the slits involves a greater degree of influence from forces acting in different directions.

Depicted in Figure 1 is a preferred embodiment of the shape of the slits 14, where the preferred combination of slits 14 has been ringed and identified with the reference designation 14c. The slits 14c contain a combination of a plurality of straight slits 14c arranged in a row having the same or a different length, where every other slit is oriented at an angle towards the previous slit, but where the slits are situated at a distance from one another. The slits 14c are arranged at an angle of between 0° and 180° relative to a longitudinally extending centre line 16a, preferably in the range from 20°-65° and/or 110°-155° in relation to the longitudinally extending centre line 16a. The diagonally extending slits 14c provide a stronger layer of material than longitudinally extending slits or laterally extending slits for a given length of the slit.

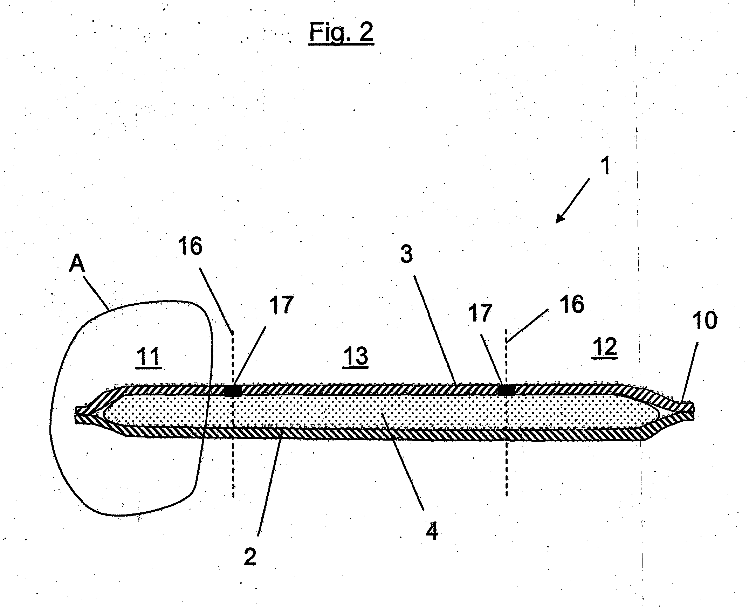

Figure 2 depicts schematically a sectional view along the line II-II in Figure 1. Figure 2 shows that the top layer 3 comprises two joining sections 17 along the lines 16 which form the boundary between the lateral zones 11, 12 and the central zone 13. The joining sections 17 join the central zone with the lateral zones and can be executed with any suitable method of joining, for example welding or gluing. The joining sections 17 are only necessary when the top layer 3 consists of two different materials, that is to say when the central zone 13 consists of a first material and the respective lateral zone 11, 12 consists of a second material. One advantage of different materials in the different zones 11, 12, 13 is that the material in the central zone 13 can be adapted to the conditions in the central zone 13, and that the material in the lateral zones 11, 12 can be adapted to the conditions in the lateral zones 11, 12. The conditions in the central zone 13 differ considerably from the conditions in the lateral zones 11, 12 because the central zone 13 is not influenced by the wearer's body movements to the same extent as the literal zones 11, 12.

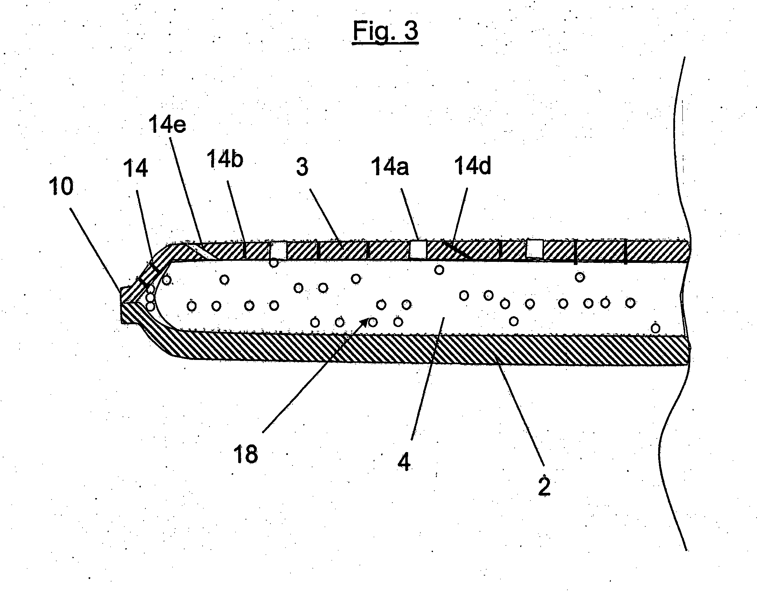

Figure 3 depicts schematically an enlarged view of section A in Figure 2. Figure 3 shows that the top layer 3 contains a number of slits 14, of which a proportion 14a are open and a proportion 14b are closed. The reason why Figure 3 shows that a proportion of slits 14a are open and a proportion of slits 14b are closed is that this symbolizes the actual condition of the top layer 3 when the lateral zones 11, 12 are influenced by the wearer's body movement when wearing the absorbent product 1. The wearer's body movement transmits a movement to the lateral zones 11, 12, which movement influences the slits 14 in such a way that they open and close. This differs from the conditions in the central zone 13, where the top layer 3 is not influenced in the same way by the wearer's body movement, but to a lesser extent, as a result of which the slits 14 do not open and close in the same way as in the lateral zones 11, 12. Depicted in Figure 3 are slits 14b cut perpendicularly to the top layer slits 14d cut at an angle to the top layer. In the latter case, an overlapping section is obtained in the slit, in that the boundary surfaces of the slit in the top layer through the angled incision have an extent in the Z-axis and in the X-axis and/or the Y-axis. The overlapping section provides a slightly better sealing ability than a perpendicular incision when the slit is closed. Depicted in Figure 3 is a partially opened, inclined slit 14e, which gives a better seal against the outward passage of material lying loosely under the top layer, at the same time as the slit permits a high transport of air.

Figure 3 also shows that the absorption body 4 comprises superabsorbents 18 (hereinafter referred to as SAP) in the form of small granules. A previously familiar problem is that SAP granules 18 migrate into the absorption body due to the movement of the lateral zones 11, 12 when the wearer moves. The SAP granules 18 may migrate out from the absorption body 4 in such a way that they lie freely under the top layer 3 in the lateral zones 11,12.

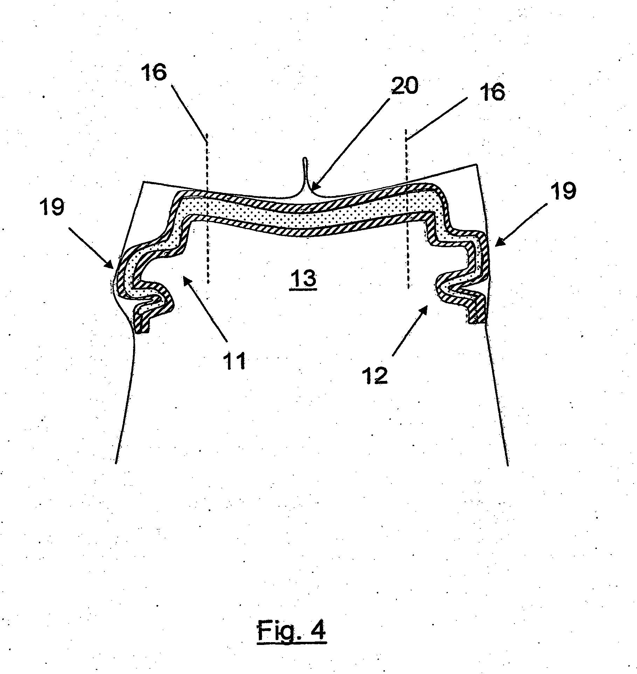

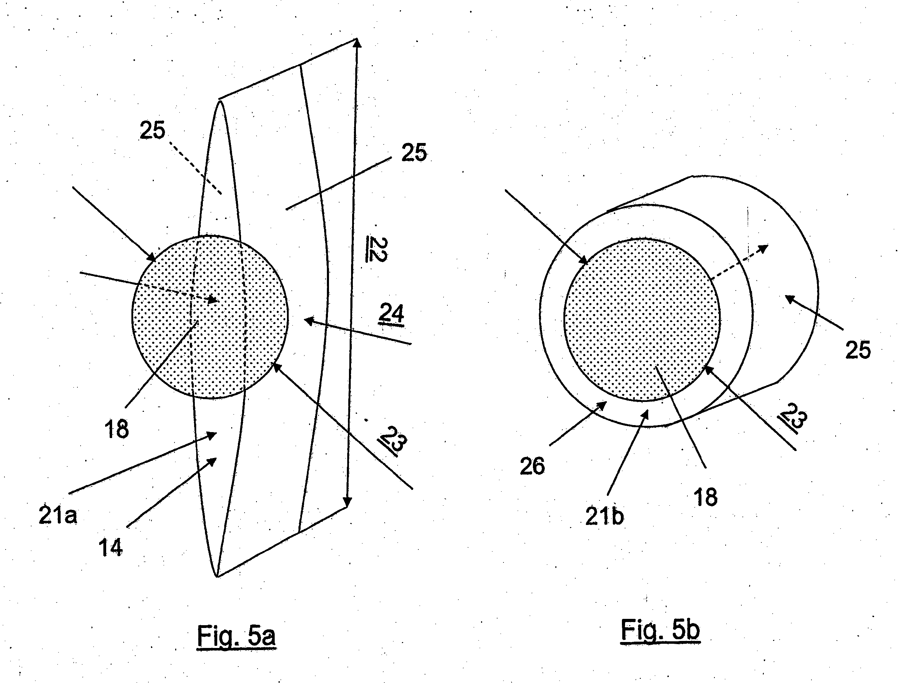

Figure 4 depicts schematically an absorbent product 1 according to Figures 1-3 positioned between the inside of the thighs 19 and against the lower abdomen 20 of a wearer. Figure 4 depicts how the absorbent product 1 has been deformed by the lateral zones 11, 12 having been folded down in relation to the central zone 13, which is attributable to the fact that the absorbent product 1 has a greater extent than the relatively plane part of the wearer's lower abdomen. Figure 4 also shows that the lateral zones 11, 12 have been deformed by the lateral zones 11, 12 having been crumpled as a result of the wearer's leg movements. It is evident from figure 4 that the central zone 13 has a more static environment than the lateral zones 11, 12. The expression static is used here to denote that the central zone 13 is less exposed to movement than the lateral zones 11, 12. The problems at the lateral zones 11, 12 referred to above thus do not arise in the same way at the central zone 13, and above all not in the central section 9, for which reason the central zone 13 of the top layer can consist of a different material than the lateral zones 11, 12 of the top layer. The lateral zones 11, 12 must be adapted in such a way that the wearer experiences the material as comfortable, which is the case with a soft material which possesses a dry feel even after the wearer has excreted fluid. The material in the lateral zones 11, 12 must be water-permeable with low reflux, furthermore, and it must prevent SAP particles 18 from finding their way out through the top layer 3 when the wearer moves. This is achieved through the invention because the lateral zones 11, 12 of the top layer 3 are embodied with slits 14 which open and close during movement. The difference between using slits and holes is illustrated in .Figures 5a and 5b below.

Figure 5a depicts schematically a slit 14 according to the invention comprising an opening 21a together with a SAP granule 18. The Figure shows that the slit 14 can be open (14a in Figure 3) without the SAP granule 18 being able to pass through the opening 21a. The slit 14 thus has the advantage that SAP granules 18 are prevented from finding their way out through the top layer 3 both when the slit 14 is closed and when the slit 14 is open. The slit 14 advantageously has a length 22 which exceeds the maximum dimension 23 of the sap granule, in which case the opening 21a can have an area greater than the maximum projected area of the SAP granule 18, but without the SAP granule 18 being able to penetrate through the opening because the width 24 of the slit is less than the minimum and maximum 23 dimensions of the SAP granule. Unlike what is depicted in Figure 5a and Figure 5b, the SAP granule 18 does not exhibit a spherical geometry, for which reason the minimum dimension 23 represents the limit for the maximum width 24 of the opening in order to ensure that the SAP granule 18 does not find its way out. It is possible for the width 24 of the opening to exceed both the minimum and the maximum dimensions 23 of the SAP granule during a short period of time, although because the slit 14 moves between the open position and the closed position, the likelihood of a SAP granule 18 leaking out is very small. The slit 14 is shown in Figure 5a to comprise two boundary surfaces 25, each delimiting the extent of the slit 14 in the longitudinal direction 22 and the thickness of the layer of material.

Figure 5b depicts schematically a hole 26 according to the prior art together with a SAP granule 18. Figure 5b depicts a SAP granule 18 of the same size as in Figure 5a, but where the opening 21b consists of a hole 26 made in the top layer 3. The hole 26 is formed by material in the top layer 3 having been removed. One effect of material having been removed is that the hole 26 is always open, but that the hole 26 can be deformed under external influence. There is very little likelihood of the hole 26 being deformed in such a way that the opening 21b is closed or becomes so small that the SAP granule 18 is not able to find its way out. If the lateral zones 11, 12 in the top layer were to be provided with holes 26 instead of slits 14, the hole 26 would always be open regardless of the movements by the wearer and the associated movements of the lateral zones 11, 12. Figure 5b shows that the SAP granule 18 is able to pass through the opening 21b and, because the hole 26 is always open, the SAP granule 18 can always pass through the opening 21b when the user is wearing the absorbent product 1. This is, in fact, how the wearer's movements increase the likelihood that SAP particles will leak out through the hole 26 in the top layer 3 because movement of the lateral zones 11, 12 causes the SAP particles 18 to move in the absorption body 4 in such a way that they end up directly adjacent to the top layer 3. The slit in Figure 5a thus possesses all the advantages mentioned above compared with the hole depicted in Figure 5b. It must also be mentioned that the SAP granules have a size of 20-400 micrometers, which means that the hole must be made smaller than this in order for the SAP granules not to be capable of finding their way out. Such small holes 26 produce a dramatic impairment of the transport of liquid from the surface layer, at the same time as which the reflux problem remains.

## Claims

1. A panty liner, sanitary towel or incontinence pad (1), having an extent in a plane in the longitudinal direction and the lateral direction and a thickness perpendicular to the plane when the product is positioned in a plane position, the panty liner, sanitary towel or incontinence pad (1) not having front, rear and side panels; the panty liner, sanitary towel or incontinence pad comprising a backing layer (2) and a top layer (3) and between them an absorption body (4), the absorption body (4) having a first surface (5) and a second surface (6), where the top layer (3) is arranged over the first surface (5) of the absorption body (4) and the backing layer is arranged over the second surface (6) of the absorption body, the panty liner, sanitary towel or incontinence pad (1) exhibiting in the longitudinal direction a rear section (7), a front section (8) and positioned between them a central section (9), and exhibiting in the lateral direction a first lateral zone (11), a second lateral zone (12) and positioned between them a central zone (13), the absorption body (4) comprising superabsorbent particles, **characterized in that** the top layer (3) comprises slits (14) only In an area which fully or partially encloses that part of the panty liner, sanitary towel or incontinence pad (1) that is formed by the section of the central zone (13) and the central section (9), and which constitutes the wet area of the product and **in that** the slits (14) have a length of 2-15 mm, preferably 3-10 mm, the slits (14) are arranged fully or partially at an angle of between 0° and 180° relative to a centre line (16a) through the panty liner, sanitary towel or incontinence pad (1) extending in the longitudinal direction, preferably in the range from 20°-65° and/or 110°-155° in relation to the centre line (16a) extending in the longitudinal direction, and the slits (14) are arranged only in the lateral zones (11, 12).

2. A panty liner, sanitary towel or incontinence pad (1) according to claim 1, **characterized in that** the slits (14) are arranged in one or both lateral zones (11, 12),

3. A panty liner, sanitary towel or incontinence pad (1) according to claim 1 or 2, **characterized in that** the slits (14) are arranged in the central section (9).

4. A panty liner, sanitary towel or incontinence pad (1) according to claim 1, **characterized in that** the slits (14) are arranged only in the central section (9).

5. A panty liner, sanitary towel or incontinence pad (1) according to any one of claims 1-3, **characterized in that** the slits (14) are arranged in the front section (8) and/or in the rear section (7).

6. A panty liner, sanitary towel or incontinence pad (1) according to any one of the preceding claims, **characterized in that** the slits (14) are straight, S-shaped, V-shaped, Z-shaped or U-shaped.

7. A panty liner, sanitary towel or incontinence pad (1) according to any one of the preceding claims, **characterized in that** the slits (14) contain a combination of a plurality of straight slits (14) arranged consecutively having the same or a different length, where every other slit is oriented at an angle to the previous slit, but where the slits are situated at a distance from one another.

8. A panty liner, sanitary towel or incontinence pad (1) according to any one of the preceding claims, **characterized in that**, on the basis of a description of the top layer (3) having an extent in a plane, one or a plurality of slits (14) is/are made in the top layer (3) through a through going incision in the top layer perpendicular to the plane.

9. A panty liner, sanitary towel or incontinence pad (1) according to any one of claims 1-7, **characterized in that**, on the basis of a description of the top layer (3) having an extent in a plane, one or a plurality of slits (14; 14d, 14e) is/are made in the top layer (3) through a through going incision in the top layer at an angle to the plane.

10. A panty liner, sanitary towel or incontinence pad (1) according to any one of the preceding claims, **characterized in that** the section of the central zone (14) and the central section (9) comprises at least one perforated two-dimensional or three-dimensional plastic or nonwoven film, where the rest of the top layer (3) comprises a slit, nonwoven in at least a part of the lateral zones (11, 12) and, where appropriate, in the front section (8) and/or in the rear section (7).

## Patentansprüche

1. Slipeinlage, Binde oder Inkontinenzeinlage (1), mit einer Erstreckung in einer Ebene in der Längsrichtung und in der Seitenrichtung und einer Dicke senkrecht zur Ebene, wenn das Produkt in einer ebenen Position positioniert ist, wobei die Slipeinlage, Binde oder Inkontinenzeinlage (1) keine Vorder-, Hinter- und Seitenelemente aufweist; wobei die Slipeinlage, Binde oder Inkontinenzeinlage eine Trägerschicht (2) und eine Oberschicht (3) und dazwischen einen Absorptionskörper (4) umfasst, wobei der Absorptionskörper (4) eine erste Oberfläche (5) und eine zweite Oberfläche (6) aufweist, wobei die Oberschicht (3) über der ersten Oberfläche (5) des Absorptionskörpers (4) und die Trägerschicht über der zweiten Oberfläche (6) des Absorptionskörpers angeordnet ist, wobei Slipeinlage, Binde oder Inkontinenzeinlage (1) in der Längsrichtung einen Hinterabschnitt (7), einen Vorderabschnitt (8) und dazwischen angeordnet einen Mittelabschnitt (9) aufweist und in der Seitenrichtung eine erste seitliche Zone (11), eine zweite seitliche Zone (12) und dazwischen angeordnet eine Mittelzone (13) aufweist, wobei der Absorptionskörper (4) superabsorbierende Partikel umfasst, **dadurch gekennzeichnet, dass** die Oberschicht (3) nur Schlitze (14) in einem Bereich umfasst, welcher ganz oder teilweise den Teil der Slipeinlage, Binde oder Inkontinenzeinlage (1) umgibt, der durch den Abschnitt der Mittelzone (13) und des Mittelabschnitts (9) gebildet ist und den Nassbereich des Produkts ausmacht, und dass die Schlitze (14) eine Länge von 2-15 mm, vorzugsweise 3-10 mm, aufweisen, wobei die Schlitze (14) ganz oder teilweise in einem Winkel von zwischen 0° und 180° im Verhältnis zu einer Mittellinie (16a) durch die Slipeinlage, Binde oder Inkontinenzeinlage (1) angeordnet sind, welche sich in der Längsrichtung erstreckt, vorzugsweise im Bereich von 20°-65° und/oder 110°-155° im Verhältnis zur Mittellinie (16a), die sich in der Längsrichtung erstreckt, und die Schlitze (14) nur in den seitlichen Zonen (11, 12) angeordnet sind.

2. Slipeinlage, Binde oder Inkontinenzeinlage (1) nach Anspruch 1, **dadurch gekennzeichnet, dass** die Schlitze (14) in einer oder beiden seitlichen Zonen (11, 12) angeordnet sind.

3. Slipeinlage, Binde oder Inkontinenzeinlage (1) nach Anspruch 1 oder 2, **dadurch gekennzeichnet, dass** die Schlitze (14) in dem Mittelabschnitt (9) angeordnet sind.

4. Slipeinlage, Binde oder Inkontinenzeinlage (1) nach Anspruch 1, **dadurch gekennzeichnet, dass** die Schlitze (14) nur in dem Mittelabschnitt (9) angeordnet sind.

5. Slipeinlage, Binde oder Inkontinenzeinlage (1) nach einem der Ansprüche 1-3, **dadurch gekennzeichnet, dass** die Schlitze (14) im Vorderabschnitt (8) und/oder im Hinterabschnitt (7) angeordnet sind.

6. Slipeinlage, Binde oder Inkontinenzeinlage (1) nach einem der vorgehenden Ansprüche, **dadurch gekennzeichnet, dass** die Schlitze (14) gerade, S-förmig, V-förmig, Z-förmig oder U-förmig sind.

7. Slipeinlage, Binde oder Inkontinenzeinlage (1) nach einem der vorgehenden Ansprüche, **dadurch gekennzeichnet, dass** die Schlitze (14) eine Kombination aus einer Mehrheit von geraden Schlitzen (14) umfassen, welche aufeinanderfolgend mit derselben oder einer anderen Länge angeordnet sind, wobei jeder zweite Schlitz in einem Winkel zum vorhergehenden Schlitz ausgerichtet ist, wobei jedoch die Schlitze mit Abstand zueinander angebracht sind.

8. Slipeinlage, Binde oder Inkontinenzeinlage (1) nach einem der vorgehenden Ansprüche, **dadurch gekennzeichnet, dass,** basierend auf einer Beschreibung der Oberschicht (3) mit einer Erstreckung in einer Ebene, ein oder eine Mehrheit von Schlitzen (14) in der Oberschicht (3) durch einen durchgehenden Einschnitt in der Oberschicht senkrecht zur Ebene bereitgestellt ist bzw. sind.

9. Slipeinlage, Binde oder Inkontinenzeinlage (1) nach einem der Ansprüche 1-7, **dadurch gekennzeichnet, dass,** basierend auf einer Beschreibung der Oberschicht (3) mit einer Erstreckung in einer Ebene, ein oder eine Mehrheit von Schlitzen (14; 14d, 14e) in der Oberschicht (3) durch einen durchgehenden Einschnitt in der Oberschicht in einem Winkel zur Ebene bereitgestellt ist bzw. sind.

10. Slipeinlage, Binde oder Inkontinenzeinlage (1) nach einem der vorgehenden Ansprüche, **dadurch gekennzeichnet, dass** der Abschnitt der Mittelzone (14) und des Mittelabschnitts (9) mindestens eine perforierte zweidimensionale oder dreidimensionale Kunststoff- oder Vliesstofffolie umfasst, wobei der Rest der Oberschicht (3) einen Schlitz, Vliesstoff in zumindest einem Teil der seitlichen Zonen (11, 12), und, wo es passend ist, im Vorderabschnitt (8) und/oder im Hinterabschnitt (7) umfasst.

## Revendications

1. Protège-slip, serviette hygiénique ou protection contre l'incontinence (1), ayant une étendue dans un plan dans la direction longitudinale et la direction latérale et une épaisseur perpendiculaire au plan lorsque le produit est placé dans une position plane, le protège-slip, la serviette hygiénique ou la protection contre l'incontinence (1) n'ayant aucun panneau frontal, arrière et latéral; le protège-slip, la serviette hygiénique ou la protection contre l'incontinence comprenant une couche de support (2) et une couche supérieure (3) et entre elles un corps absorbant (4), le corps absorbant (4) ayant une première surface (5) et une deuxième surface (6), la couche supérieure (3) étant arrangée au-dessus de la première surface (5) du corps absorbant (4) et la couche de support étant arrangée au-dessus de la deuxième surface (6) du corps absorbant, le protège-slip, la serviette hygiénique ou la protection contre l'incontinence (1) présentant dans la direction longitudinale un section arrière (7), un section frontale (8) et, située entre elles, une section centrale (9), et présentant dans la direction latérale une première zone latérale (11), une deuxième zone latérale (12) et, située entre elles, une zone centrale (13), le corps absorbant (4) comprenant des particules superabsorbantes, **caractérisé en ce que** la couche supérieure (3) ne comprend des fentes (14) que dans une région qui, entièrement ou en partie, enferme une partie du protège-slip, de la serviette hygiénique ou de la protection contre l'incontinence (1) qui est formée dans la section de la zone centrale (13) et la section centrale (9), et qui constitue la région humide du produit, et **en ce que** les fentes (14) ont une longueur comprise entre 2 et 15 mm, préférablement entre 3 et 10 mm, les fentes (14) étant arrangées entièrement ou en partie à un angle compris entre 0° et 180° par rapport à la ligne centrale (16a) à travers le protège-slip, la serviette hygiénique ou la protection contre l'incontinence (1) s'étendant dans la direction longitudinale, préférablement dans l'intervalle compris entre 20° et 65° et/ou compris entre 110° et 155° par rapport à la ligne centrale (16a) s'étendant dans la direction longitudinale, et les fentes (14) n'étant arrangées que dans les zone latérales (11, 12).

2. Protège-slip, serviette hygiénique ou protection contre l'incontinence (1) selon la revendication 1, **caractérisé en ce que** les fentes (14) sont arrangées dans l'une ou les deux zones latérales (11, 12).

3. Protège-slip, serviette hygiénique ou protection contre l'incontinence (1) selon la revendication 1 ou 2, **caractérisé en ce que** les fentes (14) sont arrangées dans la section centrale (9).

4. Protège-slip, serviette hygiénique ou protection contre l'incontinence (1) selon la revendication 1, **caractérisé en ce que** les fentes (14) ne sont arrangées que dans la section centrale (9).

5. Protège-slip, serviette hygiénique ou protection contre l'incontinence (1) selon l'une quelconque des revendications 1 à 3, **caractérisé en ce que** les fentes (14) sont arrangées dans la section frontale (8) et/ou la section arrière (7).

6. Protège-slip, serviette hygiénique ou protection contre l'incontinence (1) selon l'une quelconque des revendications précédentes, **caractérisé en ce que** les fentes (14) sont droites, en forme de S, en forme de V, en forme de Z ou en forme d'U.

7. Protège-slip, serviette hygiénique ou protection contre l'incontinence (1) selon l'une quelconque des revendications précédentes, **caractérisé en ce que** les fentes (14) contiennent une combinaison d'une pluralité de fentes droites (14) disposées consécutivement ayant la même longueur ou une longueur différente, toutes les deux fentes étant orientées selon un angle par rapport à la fente précédente, mais les fentes étant espacées l'une de l'autre.

8. Protège-slip, serviette hygiénique ou protection contre l'incontinence (1) selon l'une quelconque des revendications précédentes, **caractérisé en ce que,** sur la base d'une description de la couche supérieure (3) ayant une étendue dans un plan, une fente ou une pluralité de fentes (14) est réalisée dans la couche supérieure (3) à travers une incision traversante dans la couche supérieure perpendiculaire au plan.

9. Protège-slip, serviette hygiénique ou protection contre l'incontinence (1) selon l'une quelconque des revendications 1 à 7, **caractérisé en ce que,** sur la base d'une description de la couche supérieure (3) ayant une étendue dans un plan, une fente ou une pluralité de fentes (14; 14d, 14e) est réalisée dans la couche supérieure (3) à travers une incision traversante dans la couche supérieure à un angle au plan.

10. Protège-slip, serviette hygiénique ou protection contre l'incontinence (1) selon l'une quelconque des revendications précédentes, **caractérisé en ce que** la section de la zone centrale (14) et la section centrale (9) comprennent au moins un film non tissé ou plastique, à deux dimensions ou à trois dimensions et perforé, le reste de la couche supérieure (3) comprenant une fente non-tissée dans au moins une partie des zones latérales (11, 12) et, le cas échéant, dans la section frontale (8) et/ou dans la section arrière (7).
